# EUROPEAN PATENT SPECIFICATION

(11) **EP 2 767 543 B1**
(45) Date of publication and mention of the grant of the patent: **30.08.2017**
(21) Application number: 11874106.5
(22) Date of filing: 14.10.2011
(51) Int. Cl.: C07H 19/10, A61K 31/7068, A61P 31/18

(54) **SALTS OF CARBAMOYL PHOSPHONIC ACID ESTERS WHICH ACT AS SELECTIVE INHIBITORS OF HUMAN IMMUNODEFICIENCY VIRUS HIV-1 PRODUCTION**
SALZE AUS CARBAMOYLPHOSPHONSÄUREESTER ALS SELEKTIVE INHIBITOREN DER PRODUKTION DES HUMANEN IMMUNDEFIZIENZVIRUS HIV-1
SELS D'ESTERS D'ACIDE CARBAMOYLPHOSPHONIQUE AGISSANT COMME INHIBITEURS SÉLECTIFS DE PRODUCTION DU VIRUS D'IMMUNODÉFICIENCE HUMAINE VIH-1

(43) Date of publication of application: 20.08.2014
(73) Proprietor: Aktsionernoe Obshcestvo "Proizvodstvenno-Kommercheskaya Assotsiatsiya AZT", Moscow 121552 (RU)
(72) Inventor: GOLUBEVA, Natalya Alexandrovna, Moscow 121615 (RU); YASKO, Maxim Vladimirovich, Moscow 111396 (RU); TARUSOVA, Natalya Borisovna, Moscow 123098 (RU); BIBILASHVILI, Robert Shalvovich, Moscow 121170 (RU); KONONOV, Alexander Vasilievich, Korolev Moskovskaya obl. 141070 (RU)
(74) Representative: Friese Goeden Patentanwälte PartGmbB
(86) International application number: PCT/RU2011/000805
(87) International publication number: WO 2013/055252

(56) References cited:
- EP-A1- 1 829 885
- CA-A1- 2 504 078
- EA-B1- 014 685
- STEPHEN M BERGE ET AL: "Pharmaceutical salts", JOURNAL OF PHARMACEUTICAL SCIENCES, AMERICAN PHARMACEUTICAL ASSOCIATION, WASHINGTON, US , vol. 66, no. 1 1 January 1977 (1977-01-01), pages 1-19, XP002675560, ISSN: 0022-3549, DOI: 10.1002/JPS.2600660104 Retrieved from the Internet: URL:http://onlinelibrary.wiley.com/doi/10. 1002/jps.2600660104/abstract [retrieved on 2006-09-18]
- RICHARD J BASTIN ET AL: "salt selection and optimisation procedures for pharmaceutical new chemical entities", ORGANIC PROCESS RESEARCH AND DEVELOPMENT, AMERICAN CHEMICAL SOCIETY, US, vol. 4, no. 5, 15 September 2000 (2000-09-15), pages 427-435, XP002728118, ISSN: 1083-6160, DOI: 10.1021/OP000018U [retrieved on 2000-07-19]

## Description

### Technical Field

The invention relates to the field of molecular biology, virology and medicine and concerns new bioactive compounds, namely carbamoyl phosphonate salts. The novel compounds are able to selectively inhibit the production of human immunodeficiency virus (HIV), and can therefore be considered for use in the field of medicine for treatment of AIDS.

### Technical Background

Modified nucleosides are widely used in treatment and prevention of many viral diseases. The first Russian original anti-HIV drug - 5'-H-phosphonate 3'-azido-3'-deoxythymidine (Nikavir^{®}) - serves as an example of such a compound. Being a latent form of 3'-azido-3'-deoxythymidine, Nikavir^{®} offers several advantages over the latter: it is less toxic for patients and has a narrower range of side effects; its clearance period is two times longer, which makes it possible to reduce the dosing frequency, and a resistance to it develops much slower and at a significantly lower level.

[(2S,3S,5R)-3-azido-5-(5-methyl-2,4-deoxythymidine-1-yl)oxolan-2-yl]methyl ester of carbamoyl phosphonic acid, just like Nikavir^{®}, is a depot form of 3'-azido-3'-deoxythymidine [RU 2322450 C2. Modified 5'-Phosphonates of AZT (3'-azido-3'-deoxythymidine) as Active Components for Potential Antiviral Drugs; M.V. Ias'ko, A.V. Shipitsin, A.L. Khandazhinskaya, et al. // New derivatives of alkyl- and aminocarbonylphosphonic acids containing 3'-azido-3'-deoxythymidine. // Bioorg Khim, 2006, 32(6), 603-608; A.L. Khandazhinskaya, D.V. Yanvarev, M.V. Jasko et al. //5'-Aminocarbonyl phosphonates as new zidovudine depot forms: antiviral properties, intracellular transformations, and pharmacokinetic parameters.// Drug Metabolism and Disposition, 2009, 37(3), 494-501], however, it is extremely difficult to create its dosage form due to its instability.

### Invention Disclosure

The reported carbamoyl phosphonate salts are described using the common formula: 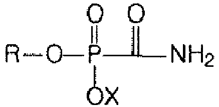 wherein
R is [(2S,3S,5R)-3-azido-5-(5-methyl-2.4-deoxypyrimidine-1-yl)oxolane-2-yl]methyl (3'-azido-3'-deoxythymidine); and
X is an alkali or alkali-earth metal ion, or an ammonium ion substituted with hydrogen; or the ammonium ion of natural alpha-amino acids and their esters; or the ammonium ion of primary, secondary and tertiary amino acids and their esters with aliphatic, aromatic, carbocyclic and heterocyclic groups; or the ammonium ion of primary, secondary and tertiary amino-alcohols with aliphatic, aromatic, carbocyclic and heterocyclic groups; or the ammonium ion of primary, secondary and tertiary amines and polymeric amines with aliphatic, aromatic, carbocyclic and heterocyclic groups, which are selective inhibitors of HIV-1 reproduction.

The reported salts are equal to the acid form [(2S,3S,5R)-3-azido-5-(5-methyl-2.4-deoxypyrimidine-1-yl) oxolane-2-yl]methyl ester of the carbamoyl phosphonic acid in terms of activity and toxicity, but, at the same time, are superior to it in terms of crystallizability and stability. Moreover, new salts expand the range of compounds with HIV-1 inhibitive activity.

The novel substances are white crystalline substances, very soluble in water, partially soluble in alcohols and almost insoluble in chloroform.

They are obtained by adding the equimolar quantity of ammonia, the appropriate amino compound or the alkali or alkali-earth metal hydroxide to the acid form of the carbamoyl phosphonate. The resultant solutions of salts are boiled down to small volumes, from which spontaneous crystallization starts, and are left at 4°C until the crystallization is complete; the precipitate is filtered and vacuum dried. The target compound yield is 95-98%.

The cleanliness and structure of new compounds has been confirmed with HPLC, UV- and NMR-spectroscopy data.

The stability analysis for the obtained compounds in the form of 1% water solutions at room temperature was carried out. The acid form of the carbamoyl phosphonate and ammonia salt degrade by 40% and 2%, respectively, within a month; no destruction products were detected for the rest of salts within a month.

The novel compounds inhibit HIV-1 reproduction in the MT-4 passaged lymphocyte culture, protecting cells from the cytopathogenic viral action, and do not demonstrate toxicity towards host cells, up to the extremely high concentrations (Table 1). The obtained experimental data suggests that the studied compounds, without having any toxic action on cells in effective concentrations (50% of the toxic dose exceeds 50% of the inhibiting doses by the factor of 2-4), suppress HIV-1 reproduction to the high extent in MT-4 cell culture. determined as the ratio of the drug's toxic dose to its effective dose, are comparable with those for the acid form of the carbamoyl phosphonate. Viral assays are conducted in accordance with the earlier described protocols.

### Best Invention Implementation Options

Below are provided the examples that reveal the invention's essence.

### Example 1

### Synthesis of the sodium salt of the carbamoyl phosphonate.

100 ml (0.5 mol) of 5 N sodium hydroxide solution are added to 187 g (0.5 mol) of solution of the acid form of the carbamoyl phosphonate and everything is stirred for 20 minutes in 350 ml of distilled water at 4°C. The resultant solution is boiled down in a rotary evaporator (bath temperature of 40°C) to 100 ml/ 150 ml volume and is left at the temperature of 4°C until the crystallization is complete. The fine-crystalline precipitate is separated using a glass filter, washed with 20 ml of water cooled to 4°C and vacuum dried. Yield: 191.2 g (96.5%).

The retention time in the Luna 100 C18(2) 150 mm x 4.6 mm column: 2.4 min (mobile phase: 0.05 M ammonia acetate solution - acetonitrile, with the 8:1 ratio, the column temperature is 40°C, the flow speed is 1 ml/min).

UV spectrum (water): λmax 266.5 nm (ε 9,800).

1N-NMR (D2O), ppm: 7.56 (1H, s, H6), 6.12 (1H, t, J 6.7 Hz, H1'), 4.38 (1H, m, H3'), 4.17 (3H, m, H4', H5'), 2.38 (2H, t, J 6.2 Hz, H2'), 1.79 (3H, s, 5-CH3).

31R-NMR (D2O), ppm: -1.42 (s).

### Example 2

### Synthesis of the potassium salt of the carbamoyl phosphonate.

100 ml (0.5 mol) of 5 N potassium hydroxide solution are added to 187 g (0.5 mol) of solution of the acid form of the carbamoyl phosphonate and everything is stirred for 20 minutes in 350 ml of distilled water at 4°C. The resultant solution is boiled down in a rotary evaporator (bath temperature: 40°C) to the volume of 100 ml/ 150 ml and is left at 4°C until the crystallization is complete. The fine-crystalline precipitate is separated using a glass filter, washed with 20 ml of water cooled down to 4°C and vacuum dried. Yield: 200.6 g (97.3%).

The retention time in the Luna 100 C18(2) 150 mm x 4.6 mm column: 2.4 min (mobile phase: 0.05 M ammonia acetate solution - acetonitrile, in the 8:1 ratio; the column temperature is 40°C, the flow speed is 1 ml/min). UV spectrum (water): λmax 267.0 nm (ε 9,600).

1N-NMR (D2O), ppm: 7.67 (1H, s, H6), 6.21 (1H, t, J 6.6 Hz, H1'), 4.50 (1H, m, H3'), 4.19 (3H, m, H4', H5'), 2.49 (2H, t, J 6.6 Hz, H2'), 1.91 (3H, s, 5-CH3).

31R-NMR (D2O), ppm: -1.59 (s).

### Example 3

### Synthesis of the calcium salt of the carbamoyl phosphonate.

1,000 ml (0.25 mol) 0.5 N of pre-filtered calcium hydroxide solution are added to 187 g (0.5 mol) solution of the acid form of carbamoyl phosphonate and everything is stirred for 20 minutes in 350 ml of distilled water at the temperature of 4°C. The resultant solution is boiled down in a rotary evaporator (bath temperature: 40°C) to the volume of 200 ml and is left at 4°C until the crystallization is complete. The fine-crystalline precipitate is separated using a glass filter, washed with 20 ml of water cooled down to 4°C and vacuum dried. Yield: 192.9 g (98.1%).

The retention time in the Luna 100 C18(2) 150 mm x 4.6 mm column is 2.4 minutes (mobile phase: 0.05 M ammonia acetate solution - acetonitrile, in the 8:1 ratio; the column temperature is 40°C, the flow speed is 1 ml/min).UV spectrum (water): λmax 266.8 nm (ε 9,400).

1N-NMR (D2O), ppm: 7.59 (1H, s, H6), 6.15 (1H, t, J 6.6 Hz, H1'), 4.41 (1H, m, H3'), 4.10 (3H, m, H4', H5'), 2.41 (2H, t, J 5.9 Hz, H2'), 1.82 (3H, s, 5-CH3).

31R-NMR (D2O), ppm: -1.59 (s).

### Example 4

### Synthesis of the barium salt of the carbamoyl phosphonate.

1,000 ml (0.25 mol) of 0.5 N pre-filtered barium hydroxide solution are added to 187 g (0.5 mol) of the acid form of carbamoyl phosphonate and everything is stirred for 20 minutes in 350 ml of distilled water at 4°C. The resultant solution is boiled down in a rotary evaporator (bath temperature: 40°C) to 300 ml and is left at 4°C until the crystallization is complete. The fine-crystalline precipitate is separated using a glass filter, washed with 20 ml of water cooled down to 4°C and vacuum dried. Yield: 216.3 g (97.9%).

The retention time in the Luna 100 C18(2) 150 mm x 4.6 mm column: 2.4 min (mobile phase: 0.05 M of ammonia acetate - acetonitrile, in the 8:1 ratio; the column temperature is 40°C, the flow speed is 1 ml/min).UV spectrum (water): λmax 266.7 nm (ε 9,400).

1N-NMR (D2O), ppm: 7.65 (1H, s, H6), 6.20 (1H, t, J 6.6 Hz, H1'), 4.47 (1H, m, H3'), 4.16 (3H, m, H4', H5'), 2.46 (2H, t, J 6.5Hz, H2'), 1.87 (3H, s, 5-CH3).

31R-NMR (D2O), ppm: -1.56 (s).

### Example 5

### Synthesis of the ammonium salt of the carbamoyl phosphonate.

100 ml (0.5 mol) of 5 N of aqueous ammonia are added to 187 g (0.5 mol) of solution of the acid form of carbamoyl phosphonate and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 186.2 g (95.2%).

The retention time in the Luna 100 C18(2) 150 mm x 4.6 mm column: 2.4 minutes (mobile phase: 0.05 M of ammonia acetate solution - acetonitrile, in the 8:1 ratio, the column temperature is 40°C, the flow speed is 1 ml/min).UV spectrum (water): λmax 266.8 nm (ε 9,650).

1N-NMR (D2O), ppm: 7.65 (1H, s, H6), 6.20 (1H, t, J 6,5 Hz, H1'), 4.46 (1H, m, H3'), 4.16 (3H, m, H4', H5'), 2.46 (2H, t, J 5.9 Hz, H2'), 1.87 (3H, s, 5-CH3).

31R-NMR (D2O), ppm: -1.59 (s).

### Example 6

### Synthesis of the L-alanine salt of the carbamoyl phosphonate.

200 ml (0.5 mol) of 2.5 N L-alanine solution are added to 187 g (0.5 mol) solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 221.5 g (95.8%).

The retention time in the Luna 100 C18(2) 150 mm x 4.6 mm column: 2.4 min (mobile phase: 0.05 M of ammonia acetate solution - acetonitrile, in the 8:1 ratio, the column temperature is 40°C, the flow rate is 1 ml/min).UV spectrum (water): λmax 266.8 nm (ε 9,600).

1N-NMR (D2O), ppm: 7.63 (1H, s, H6), 6.21 (1H, t, J 6.7 Hz, H1'), 4.43 (1H, m, H3'), 4.25 (1H, q, J 6.5 Hz, CH (alanine)), 4.10 (3H, m, H4', H5'), 2.45 (2H, t, J 6.0 Hz, H2'), 1.84 (3H, s, 5-CH3), 1.33 (3H, d, J 6.5 Hz, CH3 (alanine)).

31R-NMR (D2O), ppm: -1.57 (s).

### Example 7

### Synthesis of the ethanolammonium salt of the carbamoyl phosphonate.

100 ml (0.5 mol) of 5 N ethanolamine solution are added to 187 g (0.5 mol) solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 208.3 g (95.9%).

The retention time in the Luna 100 C18(2) 150 mm x 4.6 mm column: 2.4 min (the mobile phase: 0.05 M of ammonia acetate solution - acetonitrile, in the 8:1 ratio, the column temperature is 40°C, the flow rate is 1 ml/min).UV spectrum (water): λmax 266.8 nm (ε 9,900).

1N-NMR (D2O), ppm: 7.61 (1H, s, H6), 6.17 (1H, t, J 6.7 Hz, H1'), 4.48 (1H, m, H3'), 4.18 (3H, m, H4', H5'), 3.61 (2H, t, J 5.0 Hz, CH2O (ethanolamine)), 3.19 (2H, t, J 5.0 Hz, CH2N (ethanolamine)), 2.45 (2H, t, J 6.1 Hz, H2'), 1.86 (3H, s, 5-CH3).

31R-NMR (D2O), ppm: -1.55 (s).

### Example 8

### Synthesis of the triethanolammonium salt of the carbamoyl phosphonate.

100 ml (0.5 mol) of 5 N triethanolamine solution are added to 187 g (0.5 mol) of solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 255.5 g (97.8%).

The retention time in the Luna 100 C18(2) 150 mm x 4.6 mm column: 2.4 min (the mobile phase: 0.05 M of ammonia acetate solution - acetonitrile, in the 8:1 ratio, the column temperature is 40°C, the flow rate is 1 ml/min).UV spectrum (water): λmax 266.9 nm (ε 9,700).

1N-NMR (D2O), ppm: 7.58 (1H, s, H6), 6.15 (1H, t, J 6.5 Hz, H1') 4.39 (1H, m, H3'), 4.09 (3H, m, H4', H5'), 3.88 (6H, t, J 5.0 Hz, 3CH2O (triethanolamine)), 3.22 (6H, t, J 5.0 Hz, 3CH2N (triethanolamine)), 2.41 (2H, t, J 6.0 Hz, H2'), 1.80 (3H, s, 5-CH3).

31R-NMR (D2O), ppm: -1.53 (s).

### Example 9

### Synthesis of the 6-aminocaproic acid salt of the carbamoyl phosphonate.

200 ml (0.5 mol) of 2.5 N of 6-aminocaproic acid solution are added to 187 g (0.5 mol) of solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 232.7 g (94.9%).

The retention time in the Luna 100 C18(2) 150 mm x 4.6 mm column: 2.4 min (the mobile phase: 0.05 M of ammonia acetate solution - acetonitrile, in the 8:1 ratio, the column temperature is 40°C, the flow rate is 1 ml/min).UV spectrum (water): λmax 266.6 nm (ε 9,900).

1N-NMR (D2O), ppm: 7,64 (1H, s, H6), 6.20 (1H, t, J 6.7 Hz, H1'), 4.46 (1H, m, H3'), 4.46 (3H, m, H4', H5'), 3.02 (2H, t, J 7.1 Hz, CH2N (aminocaproic acid)), 2.45 (2H, t, J 6.1 Hz, H2'), 2.34 (2H, t, J 7.1 Hz, CH2COOH (amino capronic acid)), 1.87 (3H, s, 5-CH3), 1.52 (6H, m, 3CH2 (aminocaproic acid)).

31R-NMR (D2O), ppm: -1.57 (s).

### Example 10

### Synthesis of the pyridoxylammonium acid salt of the carbamoyl phosphonate

250 ml (0.5 mol) of 2 N pyridoxyl amine solution are added to 187 g (0.5 mol) of solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 261.2 g (96.5%).

The retention time in the Luna 100 C18(2) 150 mm x 4.6 mm column: 2.4 min (the mobile phase: 0.05 M of ammonia acetate solution - acetonitrile, in the 8:1 ratio, the column temperature is 40°C, the flow rate is 1 ml/min). UV spectrum (water): λmax 266.8 nm (ε 14,100).

1N-NMR (D2O), ppm: 8.08 (1H, s, Ar (pyridoxyl amine)), 7.65 (1H, s, H6), 6.20 (1H, t, J 6.7 Hz, H1') 4.84 (2H, s, CH2OH (pyridoxyl amine)), 4.48 (1H, m, H3'), 4.21 (2H, s, CH2NH2 (pyridoxyl amine)), 4.09 (3H, m, H4', H5'), 2.77 (3H, s, CH3 (pyridoxyl amine)), 2.47 (2H, t, J 6.1 Hz, H2'), 1.89 (3H, s, 5-CH3).

31R-NMR (D2O), ppm: -1.51 (s).

### Example 11

### Carbamoyl phosphonate and dimethylaminoethanolate salt synthesis

100 ml (0.5 mol) of 5 N dimethylaminoethanol are added to 187 g (0.5 mol) of solution of the acid form of carbamoyl phosphonate, and everything is stirred for 20 minutes in 350 ml of distilled water at 4°C. The resultant solution is boiled down in a rotary evaporator (bath temperature: 40°C) to 100 ml/ 150 ml and is left at 4°C until the crystallization is complete. The fine-crystalline precipitate is separated using a glass filter, washed with 20 ml of water cooled down to 4°C and vacuum dried. Yield: 221.2 g (95.7%).

The retention time in the Luna 100 C18(2) 150 mm x 4.6 mm column: 2.4 min (the mobile phase: 0.05 M of ammonia acetate solution - acetonitrile, in the 8:1 ratio, the column temperature is 40°C, the flow rate is 1 ml/min).UV spectrum (water): λmax 266.7 nm (ε 9,800).

1N-NMR (D2O), ppm: 7.67 (1H, s, H6), 6.24 (1H, t, J 6.7 Hz, H1') 4.49 (1H, m, H3'), 4.18 (3H, m, H4', H5'), 3.93 (2H, t, J 5.0 Hz, CH2O (dimethylaminoethanol)), 3.21 (2H, t, J 5.0 Hz, CH2N (dimethylaminoethanol)), 2.51 (6H, s, CH3 (dimethylaminoethanol)), 2.41 (2H, m, H2'), 1.89 (3H, s, 5-CH3) .

31R-NMR (D20), ppm: -1.50 (s).

### Example 12

The HIV inhibition study includes cultivation of primary infective MT-4 line lymphoid cells over the studied compounds, the ultimate concentrations of which in the culture medium range from 0.001 µg/ml to 100 µg/ml, during one passage, within 4 days.

HIV reproduction inhibition in the sensitive cell culture is determined by the reduction in p24 virus-specific protein accumulation (according to the enzyme-linked immunosorbent assay) and by the increase in cell vitality over the drug as compared with the reference cells, on Day 4 of the cultivation, using 3-(4.5-dimethylthiazole-2-yl-)-2,5-diphenyl tetrazolium (MTT) bromide staining.

### Cytotoxicity Compound Assessment

Cytotoxicity of a drug is estimated by adding its delution in RPMI-1640 serum-free medium to MT-4 cell suspension placed in cavities of the 96-cavity board (Cel-Cult, England), up to the ultimate concentrations of 0.001-100 µg/ml (three cavities per dose), with subsequent cultivation at 37°C within 4 days. The inoculation concentration is 0.5x106 cell particles in 1 mm. The cells, 100 *µ*g/ml (three cavities per dose), with subsequent cultivation at 37°C within 4 days. The inoculation concentration is 0.5x106 cell particles in 1 mm. The cells, to which the drug was not added and was substituted with the same quantity of the serum-free medium, serve as the reference. The cell vitality is counted on Day 4 of cultivation, using the formazan method (intravital staining of MTT cells). Toxicity of different drug doses is determined by the cell vitality as compared with the reference cells; the dose-dependent curve is built using obtained data, and the concentration that reduces cell vitality by 50% (CD50) is determined. The compounds in use do not have any toxic impact on MT-4 cells in effective concentrations. It is also noteworthy that 50% toxic doses exceed HIV-1 effective doses by a factor of 2-4 (see Table 1) .

The impact of the studied compounds on HIV-1 reproduction in MT-4 cell culture is studied using a well-known technique.

The therapeutic index, or the selective index (IS), is calculated as the ratio of the 50% toxic concentration of the compound to its 50% effective dose (the results are shown in Table 1). Based on these quantitative indicators of inhibition, one can judge about the efficiency of antiviral action of the reported compounds, which consists in high HIV-1 replication suppression in MT-4 cell culture, as comparable with the efficiency of the acid form of the carbamoyl phosphonate.

**Table 1. Antiviral Activity of Carbamoyl Phosphonate Salts vs HIV-1, GVK4046.**

| **Compound** | **CD₅₀, µM** | **ID₅₀, µM** | **IS** |
|---|---|---|---|
| X = Na | 2.7 | 1.2 | 2,250 |
| X = K | 2.5 | 0.9 | 2,778 |
| X = Ca | 1.9 | 1 | 1,900 |
| X = Ba | 3.5 | 1.3 | 2,692 |
| X = NH₄ | 2.7 | 0.8 | 3,375 |
| X = NH₂CH(CH₃)COOH | 2.9 | 0.9 | 3,222 |
| X = NH₂CH₂CH₂OH | 2.8 | 1.1 | 2,545 |
| X = N(CH₂CH₂OH)₃ | 3 | 0.9 | 3,333 |
| X = NH₂(CH₂)₅COOH | 3.2 | 0.8 | 4,000 |
| X = pyridoxyl amine | 2.1 | 1 | 2,100 |
| X = N(CH₃)₂CH₂CH₂OH | 2.7 | 0.9 | 3,000 |
| Acid form (reference) | 2.6 | 0.8 | 3,250 |

### Industrial Applicability

Thus, the novel substances - carbamoyl phosphonate salts - inhibit HIV-1 reproduction in lymphocyte cultures, and with approximately the same activity as was observed for the acid form of carbamoyl phosphonate. The toxicity of the reported salts is also within the toxicity of the acid form of carbamoyl phosphonate. At the same time, the novel compounds are more stable, which facilitates drug creation on their basis.

## Claims

1. Carbamoyl phosphonate salts of the general formula: 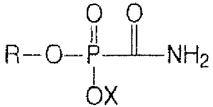 wherein
R is [(2S,3S,5R)-3-azido-S-(5-methyl-2.4-deaxypyrimidine-1-yl)oxolane-2-yl]methyl (3'-azido-3'-deoxythymidine); and
X is an alkali or alkali-earth metal ion, or an ammonium ion substituted with hydrogen; or the ammonium ion of natural alpha-amino acids and their esters; or the ammonium ion of primary, secondary and tertiary amino acids and their esters with aliphatic, aromatic, carbocyclic and heterocyclic groups; or the ammonium ion of primary, secondary and tertiary amino-alcohols with aliphatic, aromatic, carbocyclic and heterocyclic groups; or the ammonium ion of primary, secondary and tertiary amines and polymeric amines with aliphatic, aromatic, carbocyclic and heterocyclic groups, which are selective inhibitors of HIV-1 reproduction.

## Patentansprüche

1. Carbamoylphosphonatsalze der allgemeinen Formel: 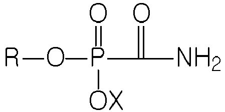 worin
R = [(2S,3S,5R)-3-azido-5-(5-methyl-2.4-desoxypyrimidin-l-yl)oxolan-2-yl]methyl (3'-azido-3'-desoxythymidin); und
X = ein Alkali- oder Erdalkali-Metallion; oder ein Ammoniumion, das mit Wasserstoff substituiert ist; oder das Ammoniumion von natürlichen α-Aminosäuren und ihren Estern; oder das Ammoniumion von primären, sekundären und tertiären Aminosäuren und ihren Estern mit aliphatischen, aromatischen, carbocyclischen und heterocyclischen Gruppen; oder das Ammoniumion von primären, sekundären und tertiären Aminoalkoholen mit aliphatischen, aromatischen, carbocyclischen und heterocyclischen Gruppen; oder das Ammoniumion von primären, sekundären und tertiären Aminen und polymeren Aminen mit aliphatischen, aromatischen, carbocyclischen und heterocyclischen Gruppen,
die selektive Inhibitoren der HIV-1 Reproduktion sind.

## Revendications

1. Sels de carbamoylphosphonate de formule général : 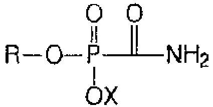 dans laquelle
R est la ((2S,3S,5R)-3-azido-5-(5-méthyl-2,4-désoxypyrimidine-1-yl)oxolane-2-yl]méthyl(3'-azido-3'-désoxythymidine) ; et
X est un ion de métal alcalin ou alcalino-terreux, ou un ion ammonium substitué par hydrogène ; ou l'ion ammonium d'acides alpha-aminés naturels et leurs esters ; ou l'ion ammonium d'acides aminés primaires, secondaires et tertiaires et leurs esters avec des groupes aliphatiques, aromatiques, carbocycliques et hétérocycliques ; ou l'ion ammonium d'amino-alcools primaires, secondaires et tertiaires avec des groupes aliphatiques, aromatiques, carbocycliques et hétérocycliques ; ou l'ion ammonium d'amines primaires, secondaires et tertiaires et d'amines polymères avec des groupes aliphatiques, aromatiques, carbocycliques et hétérocycliques, qui sont des inhibiteurs sélectifs de reproduction de VIH-1.
